# EUROPÄISCHE PATENTSCHRIFT

(11) **EP 2 519 282 B1**
(45) Veröffentlichungstag und Bekanntmachung des Hinweises auf die Patenterteilung: **19.10.2016**
(21) Anmeldenummer: 10801147.9
(22) Anmeldetag: 27.12.2010
(51) Int. Cl.: A61M 1/36

(54) **VORRICHTUNG UND VERFAHREN ZUR ÜBERWACHUNG EINER EXTRAKORPORALEN BLUTBEHANDLUNG**
DEVICE AND METHOD FOR MONITORING AN EXTRACORPOREAL BLOOD TREATMENT
DISPOSITIF ET PROCÉDÉ DE SURVEILLANCE D'UN TRAITEMENT SANGUIN EXTRACORPOREL

(30) Priorität: 28.12.2009 DE 102009060668
(43) Veröffentlichungstag der Anmeldung: 07.11.2012
(73) Patentinhaber: Fresenius Medical Care Deutschland GmbH, 61352 Bad Homburg v.d.H. (DE)
(72) Erfinder: PETERS, Arne, 61352 Bad Homburg (DE); WIKTOR, Christoph, 63571 Gelnhausen (DE)
(74) Vertreter: Oppermann, Frank
(86) Internationale Anmeldenummer: PCT/EP2010/007948
(87) Internationale Veröffentlichungsnummer: WO 2011/079941

(56) Entgegenhaltungen:
- WO-A1-00/53291
- WO-A2-2005/044339
- DE-C1- 19 848 235
- US-A- 4 739 492
- US-A- 5 091 094
- US-A1- 2001 021 817
- US-A1- 2008 217 245

## Beschreibung

Die Erfindung betrifft eine Vorrichtung zur Überwachung eines Zugangs zu einem Patienten für eine extrakorporale Blutbehandlungsvorrichtung mit einem extrakorporalen Blutkreislauf. Darüber hinaus betrifft die Erfindung eine Vorrichtung zur Überwachung eines extrakorporalen Blutkreislaufs einer extrakorporale Blutbehandlungsvorrichtung. Die Erfindung betrifft auch eine extrakorporale Blutbehandlungsvorrichtung mit einer Überwachungsvorrichtung.

Auf dem Gebiet der Medizintechnik sind verschiedene extrakorporale Blutbehandlungsvorrichtungen bekannt, die über einen extrakorporalen Blutkreislauf verfügen. Zu den bekannten extrakorporalen Blutbehandlungsvorrichtungen zählen beispielsweise Hämodialysevorrichtungen und Zellseparatoren, die einen Zugang zu dem Gefäßsystem des Patienten erforderlich machen. Bei der extrakorporalen Blutbehandlung wird dem Patienten über eine arterielle Schlauchleitung mit einer arteriellen Punktionskanüle Blut entnommen, das dem Patienten über eine venöse Schlauchleitung mit einer venösen Punktionskanüle wieder zugeführt wird.

Zum Fördern des Bluts im extrakorporalen Blutkreislauf verfügen die extrakorporalen Blutbehandlungsvorrichtungen im Allgemeinen über eine okkludierende Schlauchpumpe, insbesondere eine Rollenpumpe. Okkludierende Schlauchpumpen sind im Allgemeinen auch im Dialysierflüssigkeitssystem der extrakorporalen Blutbehandlungsvorrichtungen vorgesehen. Aus anderen medizinischen Fachgebieten, beispielsweise bei Bypass-Operationen am Herz, sind für den Betrieb eines extrakorporalen Blutkreislaufs auch andere Blutpumpen bekannt, insbesondere spezielle für Blut ausgelegte Zentrifugalpumpen, die sich durch eine verhältnismäßig geringe Blutschädigung auszeichnen.

Bei der extrakorporalen Blutbehandlung besteht trotz regelmäßiger Überwachung des Gefäßzugangs durch das Krankenhauspersonal grundsätzlich die Gefahr, dass die venöse Punktionskanüle unbemerkt aus dem Blutgefäß des Patienten herausrutscht. Während ein Herausrutschen der arteriellen Kanüle mit einem Ansaugen von Luft in die arterielle Schlauchleitung verbunden ist, führt das Herausrutschen der venösen Kanüle zu dem gefürchteten Freifluss des Blutes in die Umgebung. Wenn das Herausrutschen der venösen Kanüle daher nicht sofort erkannt wird, besteht die Gefahr, dass der Patient verblutet.

Zur Überwachung des Gefäßzugangs sind verschiedene Vorrichtungen unterschiedlicher Ausbildungen bekannt. Die bekannten Überwachungsvorrichtungen greifen im Allgemeinen auf die standardmäßig in den Blutbehandlungsvorrichtungen vorhandenen Sicherheitsvorrichtungen zurück, die bei einem nicht ordnungsgemäßen Gefäßzugang eine sofortige Unterbrechung des Blutflusses im extrakorporalen Blutkreislauf auslösen.

Aus der WO 99/29356 A1 ist eine Überwachungsvorrichtung für ein Gefäßzugang bekannt, bei der die Stärke eines elektrischen Stroms gemessen wird, der durch die Flüssigkeit in der Schlauchleitung fließt. Die US 2004/0254513 A1 beschreibt eine Überwachungsvorrichtung, bei der die Impedanz zwischen zwei an der arteriellen und venösen Schlauchleitung angeordneten Elektroden gemessen wird. Nachteilig ist, dass die bekannten Vorrichtungen die Schaffung einer elektrischen Verbindung zu der in den Schlauchleitungen strömenden Flüssigkeit erfordern.

Zur Überwachung sowohl des arteriellen als auch des venösen Gefäßzugangs sind auch Überwachungssysteme bekannt, die auf einer Messung des Drucks im extrakorporalen Blutkreislauf beruhen. Beim Herausrutschen der venösen Punktionskanüle kann in der Praxis eine Druckabnahme von 20 mmHG angenommen werden. Da der Nennwert der Messwertauflösung in der Praxis im Bereich von 2 mmHG mit einem maximalen Gesamtfehler zwischen 15 und 20 mmHG liegt, erweist sich die Detektion einer venösen Diskonnektion als schwierig. Ein Überwachungssystem mit einer Drucküberwachung ist bespielsweise aus der US 6221040 B1 bekannt. Die bekannte Drucküberwachung macht von einem speziellen Auswertverfahren Gebrauch.

Aus der WO 2006/008866 A1 und US 2005/0038325 A1 sind Überwachungsvorrichtungen bekannt, die den Austritt von Blut an der Punktionsstelle detektieren können. Diese Vorrichtungen verfügen über einen Feuchtigkeitssensor.

Die US 2008/217245 A1 beschreibt eine Blutbehandlungsvorrichtung mit einem extrakorporalen Blutkreislauf und einem Dialysierflüssigkeitssystem, bei dem Blut und Dialysierflüssigkeit mit pulsierenden Pumpen gefördert werden. Die Pumpen verfügen jeweils über einen flexiblen Pumpenraum, der von einem Druckkörper okkludiert wird.

Die US 5 091 094 A beschreibt eine Blutbehandlungsvorrichtung mit einem extrakorporalen Blutkreislauf und einem Dialysierflüssigkeitssystem. In der US 5 091 094 A ist beschrieben, dass die Dialysierflüssigkeit im Dialysierflüssigkeitssystem mit zwei Pumpen gefördert wird. Um die Blutbehandlungsvorrichtung unter geeigneten Bedingungen betreiben zu können, sollen verschiedene physikalischen Größen, wie beispielsweise die Konzentration, Temperatur, der Druck und die Flussrate, überwacht werden. Die Dialysierflüssigkeitsrate wird mit einem Durchflussmesser gemessen. Insbesondere soll der hydrostatische Druck am Dialysator überwacht und Alarm gegeben werden.

Aus der US 2001/021817 A1 ist eine Blutbehandlungsvorrichtung bekannt, die über eine peristaltische Pumpe zum Fördern von Blut im extrakorporalen Blutkreislauf verfügt, wobei eine Messeinheit zum Messen der tatsächlichen Blutflussrate vorgesehen ist. Die tatsächliche Flussrate wird mit der theoretischen Flussrate verglichen, die sich aus der vorgegebenen Drehzahl der Blutpumpe ergibt. Bei einer Abweichung wird von der Steuereinheit ein Steuersignal erzeugt, um die Drehzahl der Pumpe zu korrigieren.

Die DE 198 48 235 C1 beschreibt eine Blutbehandlungsvorrichtung mit einer peristaltischen Pumpe im extrakorporalen Blutkreislauf, bei der mittels Drucksensoren der Druck sowohl in der arteriellen als auch venösen Blutleitung gemessen wird, um den Zustand des Gefäßzugangs zu überwachen.

Aus der WO 00/53291 A ist eine Anordnung von mehreren Dialysevorrichtungen bekannt, die über eine gemeinsame Dialysierflüssigkeitszufuhr verfügen. Das Versorgungssystem für Dialysierflüssigkeit umfasst mehrere Zweige, die über Stichleitungen zu den einzelnen Dialysemaschinen führen. In der gemeinsamen Rückflussleitung wird mittels eines gemeinsamen Durchfluss-Sensors, die Rückflussrate der mittels einer gemeinsamen Pumpe geförderten Dialysierflüssigkeit gemessen.

Der Erfindung liegt die Aufgabe zu Grunde, einen Gefäßzugang und/oder einen extrakorporalen Blutkreislauf einer extrakorporalen Blutbehandlungsvorrichtung ohne umfangreiche Veränderungen an der Blutbehandlungsvorrichtung und ohne die Verwendung separater Komponenten mit besonders hoher Zuverlässigkeit zu überwachen.

Die Lösung dieser Aufgabe erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Vorrichtung beruht auf der Verwendung einer Zentrifugalpumpe zum Fördern von Blut im extrakorporalen Blutkreislauf anstelle einer okkludierenden Pumpe. Die Zentrifugalpumpe, die auch unter der Bezeichnung Kreiselpumpe bekannt ist, verfügt über ein rotierendes Pumpenrad zur Förderung von Flüssigkeiten. Die Flüssigkeit, die über das Saugrohr in die Zentrifugalpumpe eintritt, wird vom rotierenden Pumpenrad gefördert und auf einer Kreisbahn nach außen gezwungen. Die dabei aufgenommene Bewegungsenergie der Flüssigkeit erhöht den Druck innerhalb der Pumpe und presst die Flüssigkeit in das Druckrohr.

Die Erfindung macht von den besonderen Eigenschaften der bekannten Zentrifugalpumpen Gebrauch, wenn sie zur Förderung von Blut Verwendung finden. Diese Zentrifugalpumpen zeichnen sich dadurch aus, dass eine schon kleine Änderung der Druckdifferenz über der Pumpe eine große Änderung der Flussrate bewirkt.

Die erfindungsgemäße Vorrichtung verfügt über Mittel zum Messen der Flussrate des mit der Zentrifugalpumpe im extrakorporalen Blutkreislauf geförderten Bluts sowie einer Steuer- und Recheneinheit, die derart ausgebildet ist, dass bei einer Änderung der gemessenen Flussrate um mehr als einen vorbestimmten Betrag auf einen nicht ordnungsgemäßen Gefäßzugang oder eine Störung im extrakorporalen Blutkreislauf geschlossen wird. Kommt es während der extrakorporalen Blutbehandlung beispielsweise zu einem geringen Druckabfall in der venösen Blutleitung führt dies zu einem deutlichen Anstieg der Flussrate der Zentrifugalpumpe. Dieser signifikante und plötzliche Anstieg der Flussrate, die sich aus der charakteristischen flachen Förderkennlinie der Zentrifugalpumpe ergibt, wird erfindungsgemäß als Grundlage für die Erkennung eines nicht ordnungsgemäßen venösen Gefäßzugangs verwandt. Mit der Überwachung der Flussrate der Zentrifugalpumpe kann nicht nur das Herausrutschen einer der beiden Punktionskanülen aus dem Gefäßsystem des Patienten, sondern auch ein Quetschen, Knicken der Blutleitungen und eine Leckage der Schlauchleitungen erkannt werden. Die Flussrate kann grundsätzlich an einer beliebigen Stelle im extrakorporalen Blutkreislauf gemessen werden.

Von Vorteil ist, dass die erfindungsgemäße Vorrichtung nicht von externen Komponenten Gebrauch machen, die zusätzliche Handgriffe erfordern oder die Bewegungsfreiheit des Patienten unnötig einschränken.

Bei einer bevorzugten Ausführungsform der Erfindung weist die Steuer- und Recheneinheit Mittel zum Vergleichen der gemessenen Flussrate mit einer vorgegebenen Flussrate und Mittel zum Erzeugen eines Steuersignals auf, wenn die Differenz von der gemessenen Flussrate und der vorgegebenen Flussrate größer als ein bestimmter Grenzwert ist. Dieser Grenzwert ist von verschiedenen Faktoren abhängig. Beispielsweise ist der Grenzwert von der Beschaffenheit der Schlauchleitungen abhängig. In Abhängigkeit von den verschiedenen Faktoren können verschiedene Grenzwerte vorgegeben werden. Eine bevorzugte Ausführungsform sieht die Eingabe von Datensätzen auf einer Eingabeeinheit vor, die für die unterschiedlichen Faktoren, beispielsweise für das verwendete Schlauchset, charakteristisch sind. Diese Datensätze werden mit gespeicherten Datensätzen verglichen, denen bestimmte Grenzwerte zugeordnet sind, um denjenigen Grenzwert auswählen zu können, der dem eingegebenen Datensatz entspricht.

Die Überwachungsvorrichtung weist vorzugsweise eine Alarmeinheit auf, die einen akustischen und/oder optischen und/oder taktilen Alarm gibt, wenn die Steuer- und Recheneinheit das Steuersignal erzeugt.

Die Mittel zum Messen der Flussrate weisen vorzugsweise einen Flusssensor zur nicht-invasiven Messung der Blutflussrate auf. Vorzugsweise ist der Flusssensor ein Ultraschall-Flusssensor, der nach dem bekannten Ultraschall-Dopplerverfahren oder Laufzeit-Differenzverfahren die Flussrate arbeitet. Derartige Ultraschall-Flusssensoren sind dem Fachmann bekannt. Es sind aber auch alle anderen dem Fachmann bekannten Verfahren zur Messung der Flussrate einsetzbar, beispielsweise die Auswertung der Motordaten der Zentrifugalpumpe, eine magnetisch-induktive Durchflussmessung oder eine optische Durchflussmessung mit einem Laser.

Der Flusssensor kann prinzipiell an jeder Stelle im extrakorporalen Blutkreislauf, insbesondere an der arteriellen oder venösen Blutleitung angeordnet sein. Insbesondere kann der Flusssensor in die bei Blutbehandlungsvorrichtungen standardmäßig vorhandene Vorrichtung zur arteriellen Luftblasenerkennung integriert sein.

Die erfindungsgemäße Vorrichtung zur extrakorporalen Blutbehandlung verfügt über die erfindungsgemäße Überwachungsvorrichtung. Eine bevorzugte Ausführungsform der erfindungsgemäßen Blutbehandlungsvorrichtung sieht vor, dass die Steuereinheit der Blutbehandlungsvorrichtung einen Eingriff in die Maschinensteuerung vornimmt, wenn die Steuer- und Recheneinheit der Überwachungsvorrichtung ein Steuersignal erzeugt. Vorzugsweise ist die Steuereinheit derart ausgebildet, dass als Eingriff in die Maschinensteuerung die im extrakorporalen Blutkreislauf angeordnete Zentrifugalpumpe angehalten wird. Darüber hinaus wird vorzugsweise mindestens ein in oder an der venösen Blutleitung angeordnetes Absperrorgan geschlossen. Vorzugsweise wird sowohl die arterielle als venöse Schlauchklemme geschlossen. Dadurch wird bei einem nicht ordnungsgemäßen Gefäßzugang, beispielsweise wenn die venöse Punktionskanüle herausgerutscht ist oder eine Leckage im Schlauchsystem vorliegt, der Freifluss von Blut in die Umgebung sofort gestoppt.

Im Folgenden wird ein Ausführungsbeispiel der Erfindung unter Bezugnahme auf die Zeichnungen näher erläutert.

Es zeigen:
- Fig. 1: die wesentlichen Komponenten einer erfindungsgemäßen extrakorporalen Blutbehandlungsvorrichtung mit einer erfindungsgemäßen Vorrichtung zur Überwachung eines Gefäßzugangs oder des extrakorporalen Blutkreislaufs oder Dialysierflüssigkeitssystems und
- Fig. 2: der prinzipielle Verlauf der Druckdifferenz über der im extrakorporalen Blutkreislauf vorgesehenen Zentrifugalpumpe in Abhängigkeit vom Blutfluss.

Die erfindungsgemäße Vorrichtung zur Überwachung eines Gefäßzugangs oder des extrakorporalen Blutkreislaufs kann eine separate Einheit bilden oder auch Bestandteil der extrakorporalen Blutbehandlungsvorrichtung sein. Wenn die erfindungsgemäße Überwachungsvorrichtung Bestandteil der Blutbehandlungsvorrichtung ist, kann die erfindungsgemäße Überwachungsvorrichtung von bestimmten Baugruppen oder Bauteilen Gebrauch machen, die in der Blutbehandlungsvorrichtung ohnehin vorhanden sind.

Nachfolgend wird eine extrakorporale Blutbehandlungsvorrichtung A beschrieben, die über eine Vorrichtung zur Überwachung des Gefäßzugangs und des extrakorporalen Blutkreislaufs und des Dialysierflüssigkeitssystems verfügt. Es kann aber auch eine Überwachungsvorrichtung nur zur Überwachung des Gefäßzugangs oder des extrakorporalen Blutkreislaufs oder des Dialysierflüssigkeitssystems vorgesehen sein.

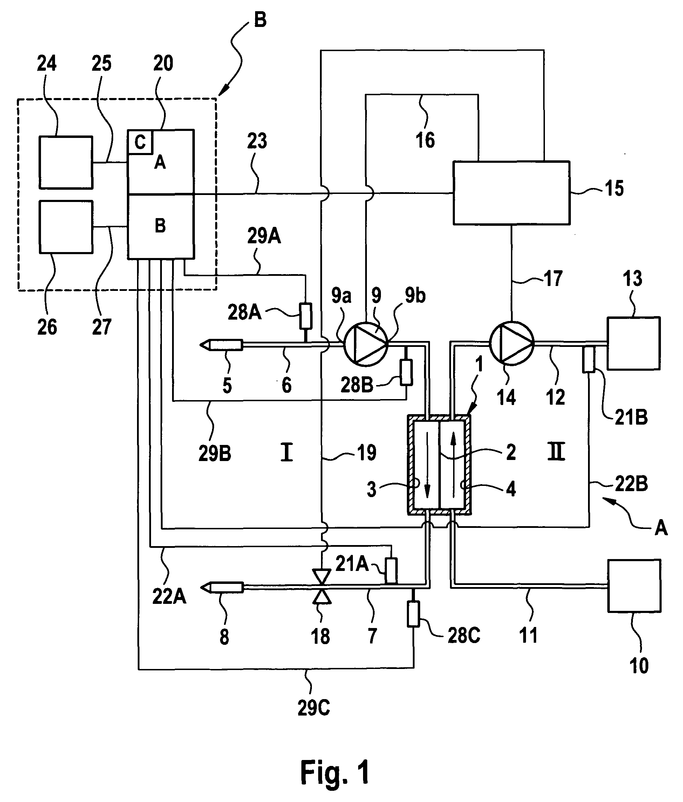

Fig. 1 zeigt nur die wesentlichen Komponenten der Blutbehandlungsvorrichtung in schematischer Darstellung, da Blutbehandlungsvorrichtungen als solche dem Fachmann bekannt sind.

Bei der Blutbehandlungsvorrichtung handelt es sich um eine bekannte Hämodialysevorrichtung, die einen Dialysator 1 aufweist, der durch eine semipermeable Membran 2 in eine Blutkammer 3 und eine Dialysierflüssigkeitskammer 4 unterteilt ist. An dem Shunt oder einer Fistel des Patienten ist mit einer arteriellen Punktionskanüle 5 eine arterielle Schlauchleitung 6 angeschlossen, die zum dem Einlass der Blutkammer 3 des Dialysators führt. Von dem Auslass der Blutkammer 3 des Dialysators geht eine venöse Schlauchleitung 7 ab, die mit einer venösen Punktionskanüle 8 an dem Shunt oder der Fistel angeschlossen ist. Das Blut wird im extrakorporalen Blutkreislauf I mit einer Zentrifugalpumpe 9 gefördert, die an der arteriellen Schlauchleitung 6 vorgesehen ist. Bei der Zentrifugalpumpe handelt es sich um eine speziell für Blut ausgelegte Pumpe, die sich durch eine geringe Blutschädigung auszeichnet. Derartige Pumpen gehören zum Stand der Technik.

Das Dialysierflüssigkeitssystem II der Hämodialysevorrichtung umfasst eine Dialysierflüssigkeitsquelle 10, an der eine Dialysierflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators geht eine Dialysierflüssigkeitsabführleitung 12 ab, die zu einem Auslass 13 führt. Die Dialysierflüssigkeit wird im Dialysierflüssigkeitskreislauf mit einer Dialysierflüssigkeitspumpe 14 gefördert, die an der Dialysierflüssigkeitsabführleitung 12 angeordnet ist. Bei dem vorliegenden Ausführungsbeispiel dient die Überwachungsvorrichtung auch der Überwachung des Dialysierflüssigkeitssystems. Daher handelt es sich auch bei der Dialysierflüssigkeitspumpe 14 um eine Zentrifugalpumpe. Dies ist aber nicht erforderlich, wenn die Überwachungsvorrichtung nur der Überwachung des Gefäßzugangs oder exktrakorporalen Blutkreislaufs dient.

Die Steuerung der Dialysevorrichtung übernimmt eine zentrale Steuereinheit 15, die über Steuerleitungen 16, 17 die Blut- und Dialysierflüssigkeitspumpe 9, 14 ansteuert. Stromab der Blutkammer 3 des Dialysators befindet sich an der venösen Schlauchleitung 7 eine elektromagnetisch betätigbare Schlauchklemme 18, die über eine weitere Steuerleitung 19 von der zentralen Steuereinheit 15 geöffnet bzw. geschlossen werden kann. Bei geschlossener venöser Schlauchklemme 18 ist die Flüssigkeitsströmung im extrakorporalen Blutkreislauf I unterbrochen, so dass Blut nicht in die Umgebung gelangen kann.

Die Überwachungsvorrichtung B verfügt über eine Steuer- und Recheneinheit 20, die in Fig. 1 als separate Einheit dargestellt ist. Die Steuer- und Recheneinheit 20 kann aber auch Bestandteil der zentralen Steuereinheit 15 der Blutbehandlungsvorrichtung sein.

Darüber hinaus verfügt die Überwachungsvorrichtung B über Mittel zum Messen der Flussrate des mit der Zentrifugalpumpe 9 im extrakorporalen Blutkreislauf I geförderten Bluts und Mittel zum Messen der Flussrate der mit der Zentrifugalpumpe 14 im Dialysierflüssigkeitssystem II geförderten Dialysierflüssigkeit. Die Mittel zum Messen der Flussrate im extrakorporalen Blutkreislauf I weisen einen Flusssensor 21 A auf, der bei dem vorliegenden Ausführungsbeispiel stromab der Dialysierflüssigkeitskammer 3 des Dialysators 1 und stromauf der Schlauchklemme 18 an der venösen Blutleitung 7 angeordnet ist, während die Mittel zum Messen der Flussrate der geförderten Dialysierflüssigkeit einen Flusssensor 21 B aufweisen, der stromab der Dialysierflüssigkeispumpe 14 angeordnet ist. Die Flusssensoren 21A und 21B sind bei dem vorliegenden Ausführungsbeispiel Ultraschall-Flusssensoren zur nicht-invasiven Messung der Flussrate des Bluts bzw. der Dialysierflüssigkeit. Die Messwerte der Ultraschall-Flusssensoren 21 A und 21 B empfängt die Steuer- und Recheneinheit 20 über Datenleitungen 22A und 22B.

Die Steuer- und Recheneinheit 20 weist Mittel 20A zum Vergleichen der gemessenen Flussrate mit einer vorgegebenen Flussrate auf. Darüber hinaus weist die Steuer- und Recheneinheit 20 Mittel 20B zum Erzeugen eines Steuersignals auf, das die zentrale Steuereinheit 15 über eine Datenleitung 23 empfängt.

Für die extrakorporale Blutbehandlung wird eine bestimmte Blutflussrate vom behandelnden Arzt vorgegeben. Die zentrale Steuereinheit 15 der Blutbehandlungsvorrichtung stellt die Drehzahl n der Zentrifugalpumpe 9 derart ein, dass das Blut im extrakorporalen Blutkreislauf I mit der vorgegebenen Flussrate gefördert wird. Die vorgegebene Flussrate wird von dem Flusssensor 21 gemessen. Diese Flussrate entspricht der Flussrate der Zentrifugalpumpe 9, die in der Steuer- und Recheneinheit 20 als Referenzwert vorgegeben wird. Daher wird diese Flussrate als vorgegebene Flussrate bezeichnet. Während der extrakorporalen Blutbehandlung wird nunmehr die Flussrate des Bluts laufend überwacht. Die mit dem Flusssensor 21 gemessene Flussrate wird mit der zuvor als Referenzwert vorgegebenen Flussrate laufend vergleichen. Es wird die Differenz zwischen der gemessenen Flussrate und der vorgegebenen Flussrate gebildet. Wenn die Differenz größer als ein bestimmter Grenzwert ist, erzeugt die Steuer- und Recheneinheit 20 das Steuersignal, das die zentrale Steuereinheit 15 über die Datenleitung 23 empfängt.

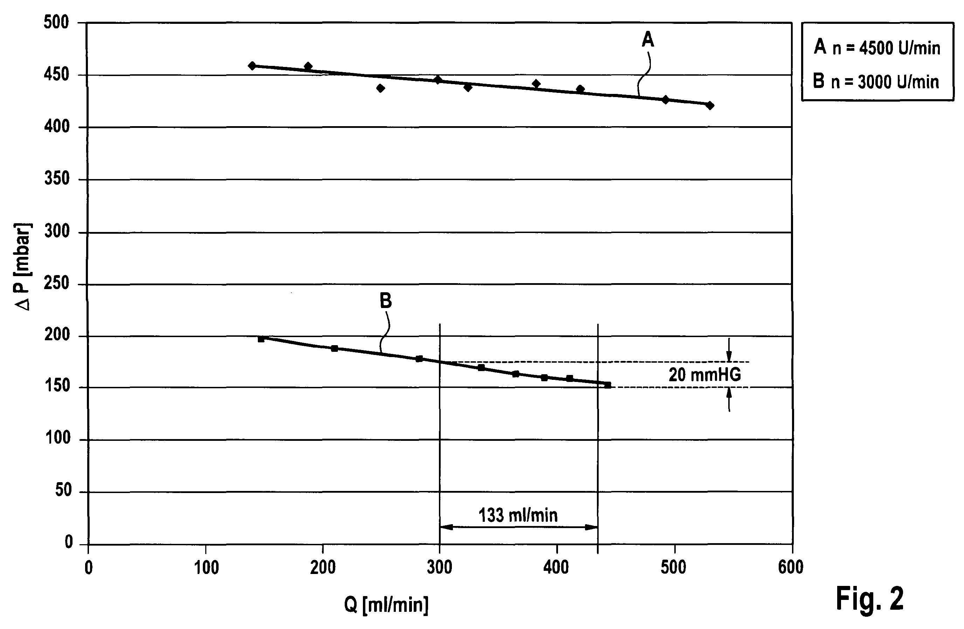

Fig. 2 zeigt die Druckdifferenz zwischen dem Einlass 9a und dem Auslass 9b der Zentrifugalpumpe 9 als Funktion der Flussrate Q. Das Herausrutschen der venösen Punktionskanüle 8 führt zu einer Druckänderung ΔP über der Zentrifugalpumpe 9 im extrakorporalen Blutkreislauf I von 20 mmHG. Fig. 2 zeigt, dass bei einer Drehzahl n von 3000 U/min die Blutflussrate Q von 300 ml/min um 133 ml/min zunimmt. Bei 4500 U/min zeigt sich eine Volumenstromzunahme von 235 ml/min. Die beiden Kennlinien (Kennlinie A 4500 U/min und Kennlinie B 3000 U/min) zeigen, dass auch eine geringe Druckänderung ΔP von 20 mmHG zu einem signifikanten Anstieg der Flussrate Q führt.

Die Steuer- und Recheneinheit 20 vergleicht die Differenz zwischen der gemessenen Flussrate und der vorgegebenen Flussrate mit einem bestimmten Grenzwert. Wenn die Differenz größer als der Grenzwert ist, d.h. ein signifikanter Anstieg der Flussrate zu verzeichnen ist, wird ein nicht ordnungsgemäßer Gefäßzugang angenommen und das Steuersignal erzeugt.

Die Überwachungsvorrichtung B weist eine Alarmeinheit 24 auf, die das Steuersignal der Steuer- und Recheneinheit 20 über eine Datenleitung 25 empfängt. Die Alarmeinheit 24 gibt dann einen akustischen und/oder optischen und/oder taktilen Alarm. Die Alarmeinheit kann aber auch Bestandteil der Blutbehandlungsvorrichtung sein. Wenn die zentrale Steuereinheit 15 der Blutbehandlungsvorrichtung das Steuersignal der Steuer- und Recheneinheit 20 empfängt, hält die zentrale Steuereinheit 15 die Zentrifugalpumpe 9 sofort an und schließt sofort die Schlauchklemme 18, so dass der Freifluss von Blut in die Umgebung sofort gestoppt wird.

Die Überwachungsvorrichtung B weist auch eine Eingabeeinheit 26 auf, die über eine Datenleitung 27 mit der Steuer- und Recheneinheit 20 verbunden ist. Die Eingabeeinheit 26 kann ebenfalls Bestandteil der Blutbehandlungsvorrichtung sein.

Auf der Eingabeeinheit 25 der Überwachungsvorrichtung B können verschiedene Parameter eingegeben werden, zu denen beispielsweise die das verwendete Schlauchsystem 6, 7 beschreibende Daten gehören, die beispielsweise den Innendurchmesser, die Wandstärke oder den Werkstoff der Schlauchleitungen angeben. Die Dateneingabe kann manuell oder automatisch erfolgen, beispielsweise mittels eines Bar-Code, Matrix-Code, RFID etc. Die Steuer- und Recheneinheit 20 weist einen Speicher 20C auf, in dem verschiedenen Datensätzen bestimmte Grenzwerte für die Überwachung des Gefäßzugangs zugeordnet sind. Die Steuer- und Recheneinheit 20 vergleicht die auf der Eingabeeinheit 25 eingegebenen Datensätze mit den zugeordneten Datensätzen und wählt den Grenzwert aus, der dem eingegebenen Datensatz entspricht. Dadurch wird sichergestellt, dass für unterschiedliche Schlauchleitungen unterschiedliche Grenzwerte für die Überwachung des Gefäßzugangs zur Verfügung gestellt werden können.

Der Grenzwert kann grundsätzlich auch dynamisch veränderlich sein. Einerseits kann der Grenzwert vom Benutzer beispielsweise vor Beginn der Behandlung fest vorgegeben werden. Andererseits kann der Grenzwert auch während der Behandlung angepasst werden. Die Anpassung kann auch automatisch erfolgen. Beispielsweise kann es aufgrund von Viskositätsänderungen des Bluts oder auch einer Änderung der Filtereigenschaften zu langsamen Flussänderungen kommen. Um in solchen Fällen keinen Alarm auszulösen kann der Grenzwert dementsprechend automatisch angepasst werden. Auch die Alarmgrenzen für abrupte Änderungen der Flussrate können bis zum Erreichen einer vorgegebenen Grenze "mitgeführt" werden. Unter "vorbestimmt" ist in diesem Zusammenhang zu verstehen, dass der Grenzwert vor seiner Verwendung in der Auswerteeinheit vorliegt bzw. bereitgestellt ist.

Die Überwachung der Flussrate der Dialysierflüssigkeit zur Erkennung einer Störung im Dialysierflüssigkeitssystem II erfolgt analog zu der Überwachung der Blutflussrate, wobei die mit dem Flusssensor 21 B gemessene Dialysierflüssigkeitsrate mit einer vorgegebenen Flussrate verglichen wird. Wenn die Differenz zwischen der gemessenen und vorgegebenen Flussrate größer als ein bestimmter Grenzwert ist, wird auf einen Störfall im Dialysierflüssigkeitssystem geschlossen. Dieser Störfall kann wieder in einer abgeknickten oder abgequetschten Schlauchleitung oder einer Leckage liegen.

Bei einer bevorzugten Ausführungform wird die Geschwindigkeit der Änderung der Flussraten in den Schlauchleitungen überwacht, wobei es sich bei den Schlauchleitungen um die arterielle und venöse Blutleitung 6, 7 oder die Dialysierflüssigkeitszuführ oder-abführleitung 11, 12 handeln kann. Dabei wird die Geschwindigkeit der Änderung der Flussrate mit einem vorgegeben Grenzwert verglichen. Auf eine Störung wird insbesondere bei einer plötzlichen Änderung der Flussarte geschlossen. Zusätzlich zu der Überwachung der Flussraten können auch die Druckverläufe in den Schlauchleitungen überwacht werden. Damit kann zwischen bestimmten Störungen, beispielsweise zwischen einer Diskonnektion einer Kanüle oder einer Leckage oder dem Verstopfen des Dialysators 1 unterschieden werden. Hierzu sind bei einer bevorzugten Ausführungsform Drucksensoren zum Messen des Drucks in den Schlauchleitungen vorgesehen.

In Fig. 1 ist ein Drucksensor 28A zum Messen des Drucks in der arteriellen Blutleitung 6 stromauf der Blutpumpe 9 und ein Drucksensor 28B zum Messen des Drucks in der arteriellen Blutleitung 6 stromab der Blutpumpe 9 sowie ein Drucksensor 28C zum Messen des Drucks in der venösen Blutleitung 7 gezeigt. Der Drucksensor 28A ist über eine Datenleitung 29A, der Drucksensor 28B ist über eine Datenleitung 29B und der Drucksensor 28C ist über eine Datenleitung 29C mit der Steuer- und Recheneinheit 20 verbunden.

Die Steuer- und Recheneinheit 20 ist bei der bevorzugten Ausführungsform derart ausgebildet, dass zwischen den nachfolgend genannten Fällen unterschieden werden kann.

Wenn die mit dem Flusssensor 21A gemessene Blutflussrate im extrakorporalen Blutkreislauf I mit einer Geschwindigkeit sinkt, die größer als ein vorgegebener erster Grenzwert ist, und der mit dem Drucksensor 28A stromauf der Blutpumpe 9 gemessene Druck unter einen vorgegeben Grenzwert sinkt, schließt die Steuer- und Recheneinheit 20 darauf, dass die Blutleitung geknickt oder gequetscht ist.

Wenn die mit dem Flusssensor 21A gemessene Blutflussrate im extrakorporalen Blutkreislauf I mit einer Geschwindigkeit sinkt, die größer als ein vorgegebener zweiter Grenzwert ist, und der mit dem Drucksensor 28B stromab der Blutpumpe 9 gemessene Druck über einen vorgegeben Grenzwert ansteigt, schließt die Steuer- und Recheneinheit 20 darauf, dass die Blutleitung geknickt oder gequetscht ist.

Wenn die mit dem Flusssensor 21A gemessene Blutflussrate im extrakorporalen Blutkreislauf I mit einer Geschwindigkeit sinkt, die größer als ein vorgegebener dritter Grenzwert ist, welcher kleiner als der oben genannte erste Grenzwert ist, d.h. die Flussrate langsamer sinkt, und der mit dem Drucksensor 28B stromab der Blutpumpe 9 gemessene Druck über einen vorgegeben Grenzwert ansteigt, schließt die Steuer- und Recheneinheit 20 darauf, dass der Dialysator 1 verstopft ist.

Wenn die mit dem Flusssensor 21A gemessene Blutflussrate im extrakorporalen Blutkreislauf I mit einer Geschwindigkeit ansteigt, die größer als ein vorgegebener vierter Grenzwert ist, d.h. die Flussrate schnell ansteigt, und der mit dem Drucksensor 28C in der venösen Blutleitung 7 gemessene Druck konstant bleibt oder fällt, schließt die Steuer- und Recheneinheit 20 darauf, dass eine Diskonnektion der venösen Kanüle 8 vorliegt.

## Patentansprüche

1. Vorrichtung zur Überwachung einer extrakorporalen Blutbehandlungsvorrichtung mit einem extrakorporalen Blutkreislauf, der eine arterielle Blutleitung mit einem arteriellen Patientenanschluss und eine venöse Blutleitung mit einem venösen Patientenanschluss aufweist, und mit einem Dialysierflüssigkeitssystem, wobei in der arteriellen oder venösen Blutleitung des extraorporalen Blutkreislaufs eine Zentrifugalpumpe zum Fördern von Blut im extrakorporalen Blutkreislauf angeordnet ist,
**dadurch gekennzeichnet, dass**
die Überwachungsvorrichtung aufweist:
Mittel (21) zum Messen der Flussrate Q des mit der Zentrifugalpumpe im extrakorporalen Blutkreislauf geförderten Bluts, und
eine Steuer- und Recheneinheit (20), die derart ausgebildet ist, dass bei einer Änderung der gemessenen Flussrate um mehr als einen vorbestimmten Betrag auf einen nicht ordnungsgemäßen Gefäßzugang geschlossen wird.

2. Vorrichtung nach Anspruch 1, **dadurch gekennzeichnet, dass** die Steuer- und Recheneinheit (20) Mittel (20A) zum Vergleichen der gemessenen Flussrate mit einer vorgegebenen Flussrate und Mittel (20B) zum Erzeugen eines Steuersignals aufweist, wenn die Differenz von der gemessenen Flussrate und der vorgegebenen Flussrate größer als ein bestimmter Grenzwert ist.

3. Vorrichtung nach Anspruch 2 **dadurch gekennzeichnet, dass** die Überwachungsvorrichtung eine Alarmeinheit (24) aufweist, die einen akustischen und/oder optischen und/oder taktilen Alarm gibt, wenn die Steuer- und Recheneinheit (20) das Steuersignal erzeugt.

4. Vorrichtung nach einem der Ansprüche 1 bis 3, **dadurch gekennzeichnet, dass** die Mittel zum Messen der Flussrate des mit der Zentrifugalpumpe im extrakorporalen Blutkreislauf geförderten Bluts einen Flusssensor (21) zur nicht-invasiven Messung der Flussrate des Bluts in der arteriellen oder venösen Blutleitung aufweisen.

5. Vorrichtung nach Anspruch 4, **dadurch gekennzeichnet, dass** der Flusssensor zur nicht-invasiven Messung der Flussrate des Bluts ein Ultraschall-Flusssensor (21) ist.

6. Vorrichtung nach einem der Ansprüche 1 bis 5, **dadurch gekennzeichnet, dass** die Überwachungsvorrichtung eine Eingabeeinheit (26) zur Eingabe von Datensätzen aufweist, wobei die Steuer- und Recheneinheit (20) einen Speicher (20C) aufweist, in dem einzelnen Datensätzen bestimmte Grenzwerte zugeordnet sind.

7. Vorrichtung zur extrakorporalen Blutbehandlung mit einem extrakorporalen Blutkreislauf (I), der eine arterielle Blutleitung (6) mit einem arteriellen Patientenanschluss (5) und eine venöse Blutleitung (7) mit einem venösen Patientenanschluss (8) aufweist, wobei in der arteriellen oder venösen Blutleitung eine Zentrifugalpumpe (9) zum Fördern von Blut im extrakorporalen Blutkreislauf angeordnet ist, **dadurch gekennzeichnet, dass** die extrakorporale Blutbehandlungsvorrichtung eine Überwachungsvorrichtung (B) nach einem der Ansprüche 1 bis 6 aufweist.

8. Vorrichtung zur extrakorporalen Blutbehandlung nach Anspruch 7, **dadurch gekennzeichnet, dass** die Vorrichtung zur extrakorporalen Blutbehandlung ein Dialysierflüssigkeitssystem (II) aufweist, das eine Dialysierflüssigkeitszuführleitung (11) und eine Dialysierflüssigkeitsabführleitung (12) aufweist, wobei in der Dialysierflüssigkeitszuführleitung oder der Dialysierflüssigkeitsabführleitung des Dialysierflüssigkeitssystems eine Zentrifugalpumpe (14) zum Fördern von Dialysierflüssigkeit angeordnet ist.

9. Vorrichtung nach Anspruch 7 oder 8, **dadurch gekennzeichnet, dass** die extrakorporale Blutbehandlungsvorrichtung eine zentrale Steuereinheit (15) aufweist, die derart ausgebildet ist, dass die Steuereinheit einen Eingriff in die Maschinensteuerung vornimmt, wenn die Steuer- und Recheneinheit (20) der Überwachungsvorrichtung ein Steuersignal erzeugt.

10. Vorrichtung nach Anspruch 9, **dadurch gekennzeichnet, dass** die Steuereinheit (15) der Blutbehandlungsvorrichtung (A) derart ausgebildet ist, dass als Eingriff in die Maschinensteuerung die im extrakorporalen Blutkreislauf (I) angeordnete Zentrifugalpumpe (9) angehalten wird.

11. Vorrichtung nach Anspruch 9 oder 10, **dadurch gekennzeichnet, dass** die Steuereinheit (15) der Blutbehandlungsvorrichtung (A) derart ausgebildet ist, dass als Eingriff in die Maschinensteuerung ein in oder an der venösen Blutleitung (7) angeordnetes Absperrorgan (18) geschlossen wird.

12. Vorrichtung nach einem der Ansprüche 7 bis 11, **dadurch gekennzeichnet, dass** die Zentrifugalpumpe (9) in der arteriellen Blutleitung (7) angeordnet ist.

## Claims

1. A device for monitoring an extracorporeal blood treatment apparatus with an extracorporeal blood circuit, which comprises an arterial blood line with an arterial patient connection and a venous blood line with a venous patient connection, and with a dialysing fluid system, wherein a centrifugal pump for conveying blood in the extracorporeal blood circuit is disposed in the arterial or venous blood line of the extracorporeal blood circuit,
**characterised in that**
the monitoring device comprises:
means (21) for measuring flow rate Q of the blood conveyed by the centrifugal pump in the extracorporeal blood circuit, and
a control and computing unit (20), which is constituted such that, in the event of a change in the measured flow rate of more than a predetermined amount, it is concluded that there is an incorrect vascular access.

2. The device according to claim 1, **characterised in that** the control and computing unit (20) comprises means (20A) for comparing the measured flow rate with a preset flow rate and means (20B) for generating a control signal when the difference between the measured flow rate and the preset flow rate is greater than a specific threshold value.

3. The device according to claim 2, **characterised in that** the monitoring device comprises an alarm unit (24), which emits an acoustic and/or optical and/or tactile alarm when the control and computing unit (20) generates the control signal.

4. The device according to any one of claims 1 to 3, **characterised in that** the means for measuring the flow rate of the blood conveyed by the centrifugal pump in the extracorporeal blood circuit comprise a flow sensor (21) for the non-invasive measurement of the flow rate of the blood in the arterial or venous blood line.

5. The device according to claim 4, **characterised in that** the flow sensor for the non-invasive measurement of the flow rate of the blood is an ultrasound flow sensor (21).

6. The device according to any one of claims 1 to 5, **characterised in that** the monitoring device comprises an input unit (26) for inputting data records, wherein the control and computing unit (20) comprises a memory (20C), in which specific threshold values are assigned to individual data records.

7. An apparatus for extracorporeal blood treatment with an extracorporeal blood circuit (I), which comprises an arterial blood line (6) with an arterial patient connection (5) and a venous blood line (7) with a venous patient connection (8), wherein a centrifugal pump (9) for conveying blood in the extracorporeal blood circuit is disposed in the arterial or venous blood line, **characterised in that** the extracorporeal blood treatment apparatus comprises a monitoring device (B) according to any one of claims 1 to 6.

8. An apparatus for extracorporeal blood treatment according to claim 7, **characterised in that** the apparatus for extracorporeal blood treatment comprises a dialysing fluid system (II) which comprises a dialysing fluid supply line (11) and a dialysing fluid discharge line (12), wherein a centrifugal pump (14) for conveying dialysing fluid is disposed in the dialysing fluid supply line or the diaiysing fluid discharge line of the dialysing fluid system.

9. The apparatus according to claim 7 or 8, **characterised in that** the extracorporeal blood treatment apparatus comprises a central control unit (15), which is constituted such that the control unit intervenes in the machine control when the control and computing unit (20) of the monitoring device generates a control signal.

10. The apparatus according to claim 9, **characterised in that** the control unit (15) of the blood treatment apparatus (A) is constituted such that the centrifugal pump (9) disposed in the extracorporeal blood circuit (I) is stopped as an intervention into the machine control.

11. The apparatus according to claim 9 or 10, **characterised in that** the control unit (15) of the blood treatment apparatus (A) is constituted such that a shut-off element (18) disposed in or on the venous blood line (7) is closed as an intervention in the machine control.

12. The apparatus according to any one of claims 7 to 11, **characterised in that** the centrifugal pump (9) is disposed in the arterial blood line (7).

## Revendications

1. Dispositif pour la surveillance d'un dispositif de traitement du sang extracorporel avec un circuit sanguin extracorporel, qui présente une conduite de sang artériel avec un raccordement artériel au patient et une conduite de sang veineux avec un raccordement veineux au patient, et avec un système de liquide de dialyse, dans lequel, dans la conduite de sang artériel ou veineux du circuit sanguin extracorporel, est agencée une pompe centrifuge pour acheminer du sang dans le circuit sanguin extracorporel,
**caractérisé en ce que**
le dispositif de surveillance présente :
des moyens (21) pour mesurer le débit Q du sang acheminé avec la pompe centrifuge dans le circuit sanguin extracorporel et
une unité de commande et de calcul (20) qui est conçue de sorte que, lors d'une modification du débit mesuré de plus d'une quantité prédéterminée, elle conclut à un accès vasculaire non conforme.

2. Dispositif selon la revendication 1, **caractérisé en ce que** l'unité de commande et de calcul (20) présente des moyens (20A) pour comparer le débit mesuré à un débit préétabli et des moyens (20B) pour produire un signal de commande lorsque la différence entre le débit mesuré et le débit préétabli est supérieure à une certaine valeur limite.

3. Dispositif selon la revendication 2, **caractérisé en ce que** le dispositif de surveillance présente une unité d'alarme (24) qui donne une alarme acoustique et/ou optique et/ou tactile lorsque l'unité de commande et de calcul (20) produit le signal de commande.

4. Dispositif selon l'une quelconque des revendications 1 à 3, **caractérisé en ce que** les moyens pour mesurer le débit du sang acheminé dans le circuit sanguin extracorporel avec la pompe centrifuge présentent un capteur de débit (21) pour la mesure non invasive du débit du sang dans la conduite de sang artériel ou veineux.

5. Dispositif selon la revendication 4, **caractérisé en ce que** le capteur de débit pour la mesure non invasive du débit du sang est un capteur de débit à ultrasons (21).

6. Dispositif selon l'une quelconque des revendications 1 à 5, **caractérisé en ce que** le dispositif de surveillance présente une unité de saisie (26) pour saisir des ensembles de données, dans lequel l'unité de commande et de calcul (20) présente une mémoire (20C) dans laquelle certaines valeurs limites sont affectées à des ensembles de données individuelles.

7. Dispositif pour le traitement du sang extracorporel avec un circuit sanguin extracorporel (I), qui présente une conduite de sang artériel (6) avec un raccordement artériel au patient (5) et une conduite de sang veineux (7) avec un raccord veineux au patient (8), dans lequel est agencée, dans la conduite de sang artériel ou veineux, une pompe centrifuge (9) pour acheminer du sang dans le circuit sanguin extracorporel, **caractérisé en ce que** le dispositif de traitement du sang extracorporel présente un dispositif de surveillance (B) selon l'une quelconque des revendications 1 à 6.

8. Dispositif pour le traitement du sang extracorporel selon la revendication 7, **caractérisé en ce que** le dispositif pour le traitement du sang extracorporel présente un système de liquide de dialyse (II), qui présente une conduite d'alimentation en liquide de dialyse (11) et une conduite d'évacuation de liquide de dialyse (12), dans lequel, dans la conduite d'alimentation en liquide de dialyse ou dans la conduite d'évacuation du liquide de dialyse du système de liquide de dialyse, une pompe centrifuge (14) est agencée pour acheminer le liquide de dialyse.

9. Dispositif selon la revendication 7 ou 8, **caractérisé en ce que** le dispositif de traitement du sang extracorporel présente une unité de commande centrale (15) qui est conçue de sorte que l'unité de commande effectue une intervention dans la commande machine lorsque l'unité de commande et de calcul (20) du dispositif de surveillance produit un signal de commande.

10. Dispositif selon la revendication 9, **caractérisé en ce que** l'unité de commande (15) du dispositif de traitement du sang (A) est conçue de sorte que soit maintenue, en tant qu'intervention dans la commande machine, la pompe centrifuge (9) agencée dans le circuit sanguin extracorporel (I).

11. Dispositif selon la revendication 9 ou 10, **caractérisé en ce que** l'unité de commande (15) du dispositif de traitement du sang (A) est conçue de sorte que soit fermé, en tant qu'intervention dans la commande machine, un organe d'arrêt (18) aménagé dans ou sur la conduite de sang veineux (7).

12. Dispositif selon l'une quelconque des revendications 7 à 11, **caractérisé en ce que** la pompe centrifuge (9) est agencée dans la conduite de sang artériel (7).
